# EUROPEAN PATENT APPLICATION

(11) **EP 3 192 533 A1**
(43) Date of publication of application: **19.07.2017**
(21) Application number: 15839965.9
(22) Date of filing: 07.09.2015
(51) Int. Cl.: A61L 27/00, C12N 5/071

(54) **METHOD OF PRODUCING FULL THICKNESS SKIN HAVING SKIN ACCESSORY ORGANS**

(30) Priority: 08.09.2014 JP 2014182611
(71) Applicant: ORGAN TECHNOLOGIES, INC., Tokyo 105-0001 (JP)
(72) Inventor: TSUJI, Takashi, Ashiya-shi Hyogo 659-0095 (JP); TOYOSHIMA, Koh-ei, Tokyo 108-0074 (JP)
(74) Representative: Weickmann & Weickmann PartmbB
(86) International application number: PCT/JP2015/075287
(87) International publication number: WO 2016/039279

(57) **Abstract**

The present invention provides a method for efficiently manufacturing full-thickness skin with skin appendage. The present invention provides a method for manufacturing full-thickness skin with skin appendage, characterized in that said method comprises the following steps (a) - (d): (a) a step of stimulating an embryoid body with a bioactive substance that may activate the Wnt pathway, (b) a step of preparing a conjugate comprising all or a part of said embryoid body stimulated in step (a) and a scaffolding material, (c) a step of transplanting said conjugate prepared in said step (b) to an animal, and (d) a step of manufacturing full-thickness skin derived from said conjugate in said animal.

## Description

### Technical Field

The present invention relates to a method for manufacturing full-thickness skin with skin appendage, as well as the manufactured full-thickness skin with skin appendage.

### Background Art

Pharmacological/safety tests employing mice and rats etc. are performed in the development of cosmetics or skin pharmaceuticals. However, in recent years, the development of an alternative method for animal experiments is searched worldwide in view of animal welfare.

Although alternative test methods employing cultured cells or a cell sheet mimicking the epidermis or dermis of animals have been developed as alternative methods for animal experiments, these alternative skin do not comprise skin appendages that the animal skin has (such as hair follicles, nails, sebaceous glands, sweat glands, and mammary glands). Alternative skin without skin appendages has poor skin barrier function because there is no secretion of sebum or sweat etc., and it was difficult to obtain test results close to those in a living body. Moreover, test results in regards to the influence of cosmetics or pharmaceuticals on skin appendages themselves also could not be obtained with alternative skin without skin appendages.

As a method for manufacturing an artificial skin with skin appendages, for example a method for co-culturing fat-derived stem cells, epidermal keratinocytes, and fibroblasts at the dermabrasion site of e.g. a nude mouse has been attempted (Patent Literature 1).

### Citation List

[Patent Literature 1] Japanese Published Unexamined Patent Application Publication No. 2009-11588

### Summary of the Invention

### Problems to be Solved by the Invention

For example, in a method such as that described in the above Patent Literature 1, although skin appendage is induced partially, the induced skin appendage will be united and inseparable with nude mouse skin, and it was thus difficult to obtain stem cell-derived full-thickness skin (i.e. skin tissue comprising at least the epidermal layer, the dermal layer, and the subcutaneous tissue) comprising skin appendage. The present inventors focused on this technical problem to attempt to develop a method for efficiently manufacturing full-thickness skin with skin appendage derived purely from the individual of interest.

### Means for Solving the Problems

As a result of repeated investigation to solve the above problems, the present inventors found that by stimulating an embryoid body with a bioactive substance that may activate the Wnt pathway, and allowing it to bind with a scaffolding material and then transplanting it to an animal, full-thickness skin with skin appendage can be efficiently manufactured, thus arriving at the completion of the present invention.

In other words, the present invention provides a method for manufacturing full-thickness skin with skin appendage, characterized in that
said "full-thickness skin with skin appendage" comprises at least the following (1) - (3):
(1) skin comprising epidermal and dermal layers,
(2) at least one type of skin appendage, and
(3) subcutaneous tissue,
   wherein said method comprises the following steps:
   (a) a step of stimulating an embryoid body with a bioactive substance that may activate the Wnt pathway,
   (b) a step of preparing a conjugate comprising the following (A) and (B):
      (A) all or a part of said embryoid body stimulated in step (a) and
      (B) a scaffolding material
   (c) a step of transplanting said conjugate prepared in said step (b) to an animal, and
   (d) a step of manufacturing full-thickness skin derived from said conjugate in said animal.

Moreover, one embodiment of the present invention is characterized in that said animal is a non-human animal.

Moreover, one embodiment of the present invention is characterized in that said non-human animal is a non-human immunodeficient animal.

Moreover, one embodiment of the present invention is characterized in that said Wnt pathway is the classical Wnt pathway.

Moreover, one embodiment of the present invention is characterized in that said "bioactive substance that may activate the Wnt pathway" is selected from a group consisting of Wnt1, Wnt2, Wnt2b, Wnt3, Wnt3a, Wnt6, Wnt7b, Wnt8a, Wnt8b, Wnt10b, and TGF-β. Moreover, said "bioactive substance that may activate the Wnt pathway" may be e.g. a Wnt receptor agonist.

Moreover, one embodiment of the present invention is characterized in that said embryoid body is an embryoid body created from an iPS or ES cell.

Moreover, one embodiment of the present invention is characterized in that said scaffolding material is a collagen gel.

Moreover, one embodiment of the present invention is characterized in that said transplantation is transplantation to the subrenal capsule.

In another embodiment of the present invention, full-thickness skin with skin appendage manufactured by any of the methods above is provided.

Needless to say, any combination of one or more characteristics of the present invention above is encompassed by the present invention.

### Effects of the Invention

According to the method for manufacturing the full-thickness skin according to the present invention, full-thickness skin with skin appendage can be manufactured with high incidence in a pluripotent stem cell-derived teratoma. Since the full-thickness skin manufactured by the method of the present invention has functional skin appendage similarly to a living animal body, it can for example be favorably employed for pharmacological or safety tests of cosmetics or pharmaceuticals. Moreover, for example, by manufacturing the full-thickness skin of the present invention with pluripotent stem cells derived from various individuals (such as derived from individuals of differing race, color of skin, age, sex, and the like), appropriate pharmacological or safety tests according to the target of the cosmetics or pharmaceuticals can be performed.

Moreover, the full-thickness skin manufactured by the method of the present invention has extremely low risk of causing a tumor by transplantation to animal skin, and thus can also be favorably employed for transplantation to a living body.

### Brief Description of the Drawings

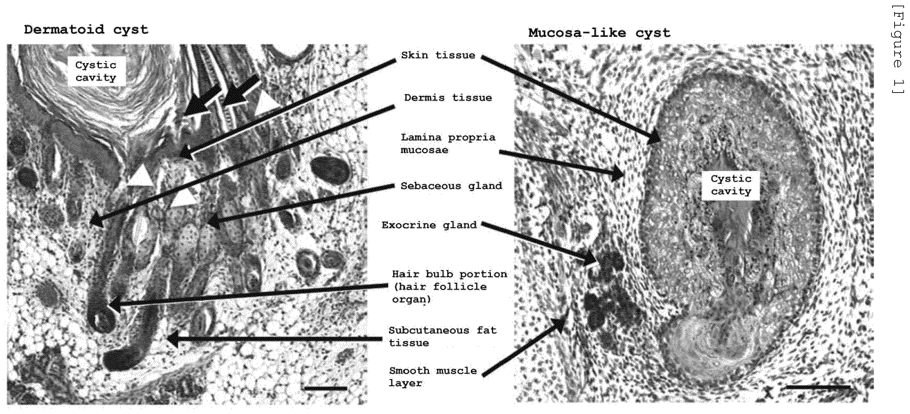

Figure 1 shows the HE staining image of a teratoma that was formed by *in vivo* transplantation of multiple embryoid bodies, which were subjected to Wnt10b stimulation. The left figure shows a dermatoid tissue comprising ectodermal cyst hair follicles and the right figure shows a mucosa-like tissue.

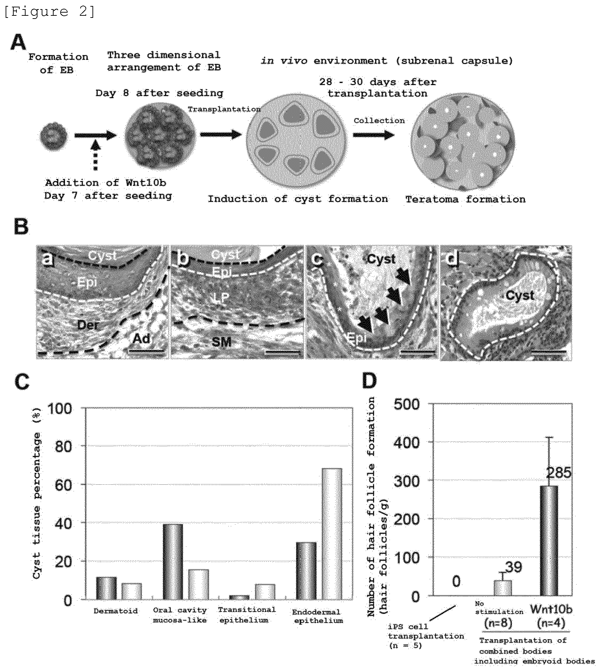

Figure 2A shows the schematic diagram of the method for forming teratomas encapsulating various organs which is one embodiment of the present invention. Embryoid bodies were formed from iPS cells, arranged three dimensionally in a collagen gel, and then transplanted in vivo to form teratomas. Moreover, Wnt10b stimulation was performed from Day 7 of embryoid body formation for 24 hours, and then three dimensional arrangement was performed. Figure 2B shows the classification of various organs formed in teratomas. a - d in each figure shows typical tissue images of a: dermatoid tissue, b: mucosa-like tissue, c: cyst tissue consisting of transitional epithelium, and d: cyst tissue consisting of endodermal epithelium, respectively. Each denotation in the figures show Cyst: cystic cavity, Epi: epithelial tissue, Der: dermis tissue, Ad: fat tissue, LP: lamina propria mucosae, Sand M: smooth muscle tissue, respectively. The cystic epithelial tissue in c is configured from simple columnar epithelium comprising squamous stratified epithelium (*) and goblet cells (arrow). Figure 2C shows a graph that compares incidences of organs induced with teratomas derived from embryoid bodies subjected to Wnt10b stimulation (dark bars) and incidences of organs induced with teratomas derived from embryoid bodies without Wnt10b stimulation (light bars). Figure 2D shows a graph that compares the number of hair follicle formation induced per 1 g of teratomas derived from multiple embryoid bodies subjected to Wnt10b stimulation (dark bar) and the number of hair follicle formation induced per 1 g of teratomas derived from multiple embryoid bodies without Wnt10b stimulation (light bar).

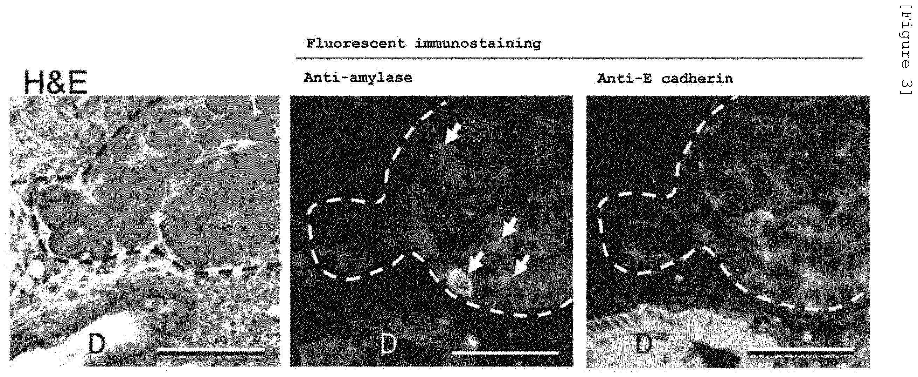

Figure 3 shows the tissue image of a secretory gland-like structure induced by teratomas derived from embryoid bodies subjected to Wnt10b stimulation. The left figure shows the HE staining image, the center figure shows the fluorescent immunostaining image by anti-amylase antibodies, and the right figure shows the immunostaining image by anti-E cadherin antibodies. Arrows in the figure show the granular amylase in the cytoplasm in the acinus-like tissue. The range of the broken lines in the figures show the range of the acinar tissue, and "D" in the figures show the proximal vessel-like structures.

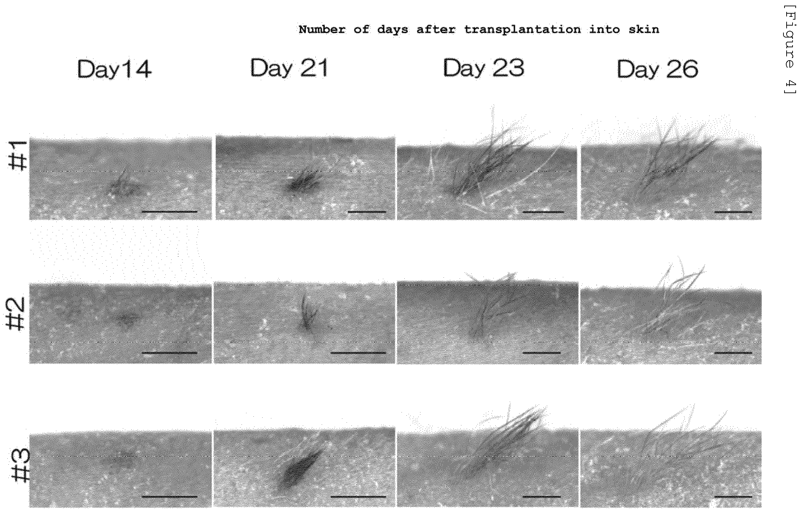

Figure 4 is photographs showing the time lapse after transplantation of full-thickness skin comprising iPS-induced hair follicles to nude mice. Dermatoid cysts comprising hair follicles induced from iPS cells were separated, cut up into full-thickness skin comprising about 20 hair follicles and transplanted to the dorsal skin of nude mice, and the hair shaft growth after transplantation was observed over time. The photographs show tracking examples of hair shafts grown from full-thickness skin comprising hair follicles transplanted at 3 locations.

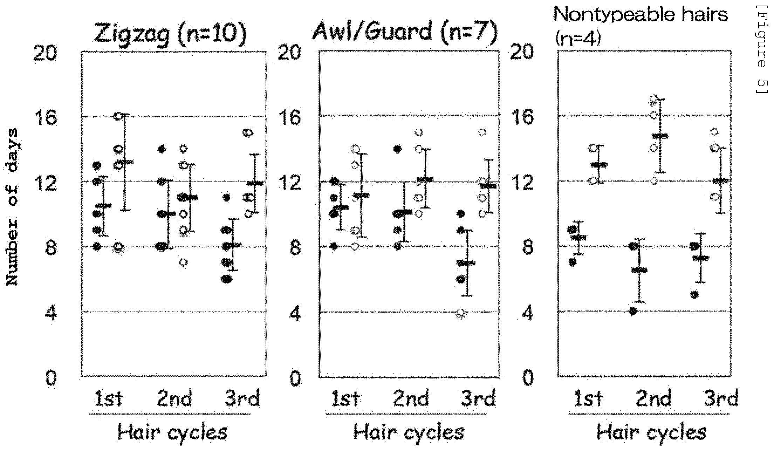

Figure 5 is figures showing the analysis of the hair cycle of hair derived from iPS-induced hair follicles. The hair shaft growth of hair follicles induced from iPS cells was observed over time. The pores of growing hair were identified, and the number of days for hair growth period as well as hair growth rest and shedding periods of the first to third hair cycles were counted. Hair type (described as Zigzag hair, Awl/Guard hair, and "nontypeable hairs" for hair that cannot be identified by morphological definition of body hair) was distinguished from the magnified photograph at the maximum growing phase of the tracked hair. Black circles show the number of days of the period that hair shafts are growing, and white circles show the number of days of the period that hair growth is resting and shedding.

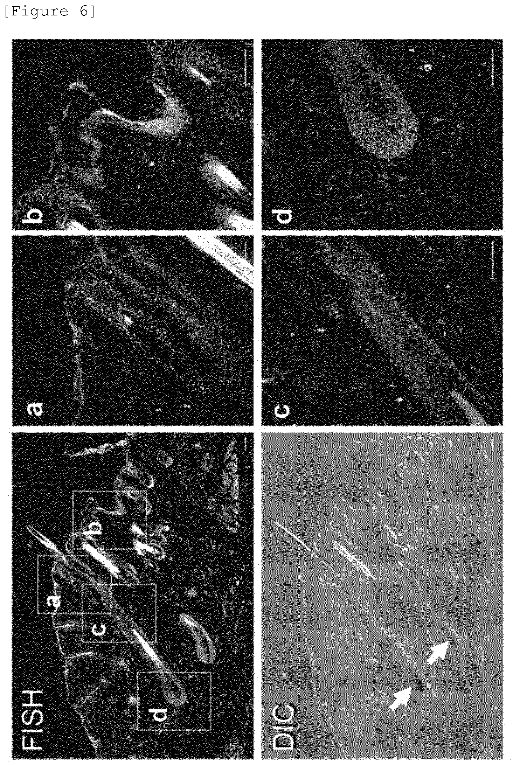

Figure 6 shows the results of Y chromosome-labeled fluorescent *in situ* hybridization (FISH) on tissue developed from the transplantation site of iPS-induced hair follicles. The figure described as "FISH" shows the low magnification image of Y chromosome FISH of the whole tissue area of transplantation to nude mice as well as the surrounding host skin. The figure described as "DIC" is the differential interference image of the tissue shown in FISH, and the arrows show iPS-induced hairs with melanin pigmentation. In the low magnification image of FISH, the tissue regions are shown by white rectangles, and the magnified photographs are shown in a - d. The white dots in the figure are the Y chromosomes detected by FISH.

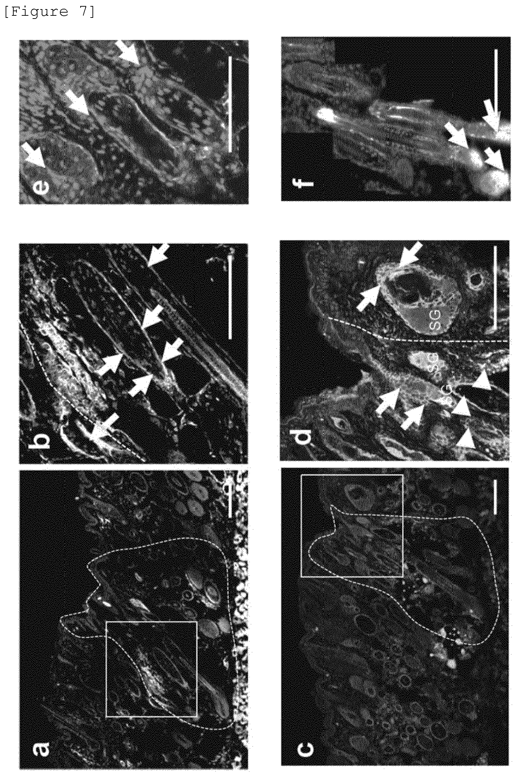

Figure 7 is immunostaining images showing that stem cell niche is reproduced in tissue developed from the transplantation site of iPS-induced hair follicles. Figures 7a and 7b shows the double immunostaining images by anti-CK15 and anti-CD34 antibodies which are hair follicle epithelium stem cell markers. Figure 7a is the low magnification image of full-thickness skin comprising iPS-induced hair follicle transplantation site, and the area framed by dotted line shows the transplanted tissue. The magnified image of the area framed by the white rectangle in Figure 7a is shown in Figure 7b. The arrows in Figure 7b show the hair follicle outer root sheaths co-stained by anti-CK15 and anti-CD34 antibodies. Figures 7c and 7d show immunostaining images against sebaceous gland progenitor cell marker Lrig1 and epithelial stem cell marker CK15. The area of the dotted line in Figure 7c represents the transplanted tissue. The magnified photograph of the area framed by the white rectangle in Figure 7c is shown in Figure 7d, and Lrig1-positive cells are shown with arrows and CK15-positive cells are shown with arrowheads. In the figure, SG represents sebaceous glands. Figure 7e shows the immunostaining image employing the anti-Lgr6 antibody. Lgr6 is expressed at the outer root sheaths (arrows) near the sebaceous gland attachment site. Figure 7f shows the immunostaining image employing the anti-Lgr5 antibody. Lgr5 is recognized to be expressed at variable regions of the growing phase hair follicles, and in particular strongly expressed in epithelial cells near the hair matrix region (arrows).

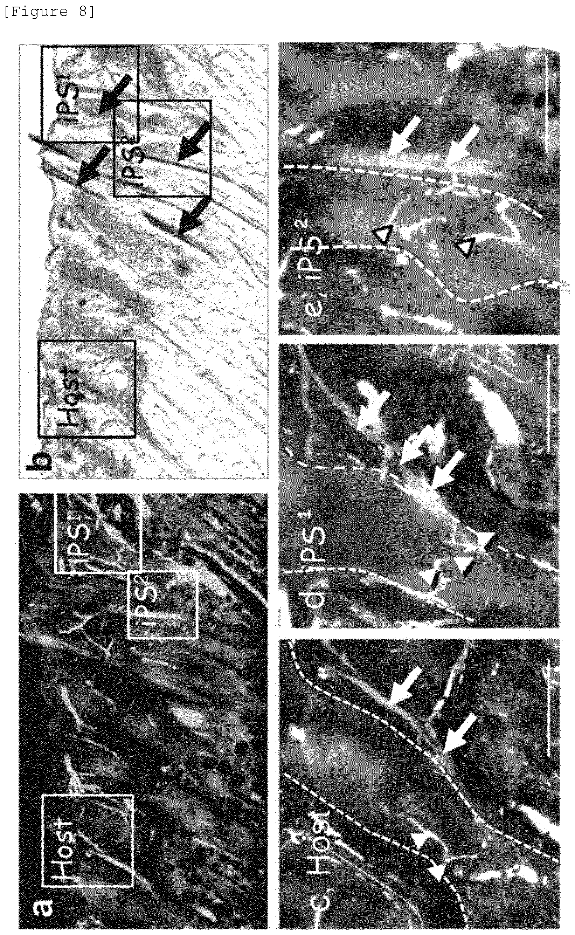

Figure 8 is figures showing that iPS-induced hair follicles are connected with arrector pili muscles and nerves in a tissue developed from the transplantation site of iPS-induced hair follicles. Full-thickness skin comprising hair follicles derived from iPS were transplanted to the dorsal portion of nude mice, and tissues were collected when the iPS cell-derived hair follicles entered the third growing phase to create tissue sections with a thickness of 100 µm. The fluorescent immunostaining image by antibodies that recognize smooth muscle marker calponin and nerve fiber marker neurofilament H (NF-H) (Figure 8a), and the stereomicroscopic image of the same section by transmitted light (Figure 8b) are shown, respectively. Hair follicles where hair shaft possessing melanin pigment was observed in the hair follicle by stereomicroscopic imaging (Figure 8b, black arrows) show that they are derived from iPS cells. Within the white rectangles of the low magnification image, the magnifications of host hair follicles (Figure 8c, Host) and areas comprising iPS cell-derived hair follicles (Figure 8d, iPS 1 and Figure 8e, iPS 2) are shown in c - e. Calponin-positive arrector pili muscles (white arrows) and NF-H-positive nerve fibers (white arrowheads) connected to each hair follicle are shown.

### Description of Embodiments

"Pluripotent stem cells" as used herein refer to cells having both the differentiation versatility of being able to differentiate into any and all cell types of the living body and the self-replication ability of being able to maintain the differentiation versatility even after going through proliferation and differentiation, examples of which include ES cells or iPS cells.

"ES cells (Embryonic Stem cells)" as used herein refer to a stem cell strain that is created from an inner cell mass that belongs to a part of an embryo in the blastocyst stage which is the early development of an animal, which has the differentiation versatility of being able to differentiate into a great many number of cells as well as the self-replication ability of maintaining the differentiation versatility even after going through division and proliferation.

The origin of the ES cell that can be employed in the present invention is not particularly limited, and an ES cell derived from an inner cell mass of any and all animals can be employed. For example, as the origin of an ES cell, an ES cell derived from an inner cell mass of a human, a mouse, a rat, a pig, or a monkey can be employed.

"iPS cells (induced Pluripotent Stem cells)" as used herein refer to a cell which is rendered the differentiation versatility of being able to differentiate into a great many number of cells as well as the self-replication ability of maintaining the differentiation versatility even after going through division and proliferation as with an ES cell by introducing e.g. several types of genes and/or agents into a somatic cell.

The origin of the iPS cell that can be employed in the present invention is not particularly limited, and an iPS cell derived from any and all animals can be employed. For example, as the origin of an iPS cell, an iPS cell derived from a human, a mouse, a rat, a pig, or a monkey can be employed. Moreover, the somatic cell to be the origin of the iPS cell that can be employed in the present invention is also not particularly limited, and an iPS cell induced from a cell derived from any and all tissues can be employed. Further, the method for inducing the iPS cell that can be employed in the present invention is also not particularly limited, and an iPS cell induced by any method can be employed as long as it is a method that can induce an iPS cell from a somatic cell.

In the present invention, the method for culturing pluripotent stem cells without differentiating them is not particularly limited, and a culture environment or medium well-known to those skilled in the art, or a culture environment or medium in accordance thereto can be appropriately selected. For example, mouse embryonic fibroblasts (MEF) can be employed as the feeder cells for culturing pluripotent stem cells. Moreover, a medium generally employed for culturing pluripotent stem cells can be employed as the medium for culturing pluripotent stem cells, the composition of which is not particularly limited.

A "teratoma" as used herein is a highly differentiated germ cell tumor having a diploblastic or triploblastic component, and is also referred to as a teratoid tumor. A "teratoma" as used herein includes structures that histologically resemble a teratoid tumor that may be produced when pluripotent stem cells are transplanted to a living body. Although a teratoma is sometimes naturally produced *in vivo,* it can also be artificially produced by transplanting pluripotent stem cells into an animal.

In the present invention, the site for transplanting the cells into an animal is not particularly limited, and for example, transplantation can be made to the subrenal capsule, the subcutaneous, or the testis of an animal.

In the present invention, the method for transplanting the cells into an animal is not particularly limited, and for example, when transplanting into the subrenal capsule of a mouse, an incision of 2 - 3 mm is made in the renal capsule, the renal capsule and the renal parenchymal are detached, and the cells can be transplanted therebetween.

In the present invention, the method for confirming that a teratoma is formed in an animal having pluripotent stem cells transplanted is not particularly limited, and for example, confirmation can be made by performing a laparotomy 3 - 4 weeks after transplantation and visually confirming tumor mass formation by appearance. Preferably, triploblastic tissue formation which is characteristic of a teratoma can be confirmed by histologically analyzing the tumor mass.

A "full-thickness skin" as used herein refers to a laminar tissue structure comprising at least the following (1) - (3).
(1) skin comprising epidermal and dermal layers,
(2) at least one type of skin appendage, and
(3) subcutaneous tissue.

A "skin appendage" as used herein means, but is not limited to, the corneal organs and the exocrine gland which are connected via the epidermal layer and the epithelial tissue that configure the skin, as well as have inherent functions. A skin appendage as used herein means, but is not limited to, for example organs distributed in the skin such as hair follicles, nails, sebaceous glands, sweat glands, and mammary glands.

A "subcutaneous tissue" as used herein is a tissue that supports the skin and skin appendages as well as binds these with other organ lines, the examples of which include, but are not limited to, the subcutaneous fat tissue, the panniculus carnosus configured by the smooth muscle tissue, and the connective tissue configured by collagen fibers or elastic fibers.

An "organ primordium" as used herein refers to the area of an embryo or the structure of an embryo that is determined to develop into a particular organ with the progression of the development stage *in vivo,* and is sometimes referred to simply as a "primordium." Almost all organs in the living body are developed from the organ primordiums induced from the epithelial line stem cells and the mesenchymal lineage stem cells by the development program in the fetal stage to develop into given positions and given numbers.

In the present invention, the method for confirming that the full-thickness skin of interest is formed in the teratoma is not particularly limited, and for example, confirmation can be made by dissecting the teratoma, and searching from appearance the structure that is believed to be the full-thickness skin with skin appendage (such as hair follicles, nails, sebaceous glands, sweat glands, and mammary glands). Preferably, teratoma tissue sections are created, and identification of a given organ can be confirmed from the tissue structure. When a more detailed confirmation is to be made, whether genes to be expressed at each organ are expressed at appropriate sites can be analyzed by an *in situ* hybridization method.

An "embryoid body" as used herein refers to the cell mass formed when pluripotent stem cells such as ES cells or iPS cells are cultured in suspension. An embryoid body may take an embryoid form, and may be composed of various tissues. The method for creating the embryoid body that can be employed in the present invention is not particularly limited, and for example, the method of seeding pluripotent stem cells in a low adherence plate, the hanging drop method of hanging cell suspension droplets of pluripotent stem cells, and the method of culturing a culture dish of pluripotent stem cells in suspension with shaking can be employed.

For example, when embryoid bodies are created with the method of seeding iPS cells in a low adherence plate, embryoid bodies can be created by seeding and culturing iPS cells at 1500 cells - 10000 cells/200 µl/well and more preferably 2000 cells - 4000 cells/200 µl/well in a 96-well low adherence plate. When the seeded cells are less than 1500 cells/200 µl/well, there is a risk that embryoid bodies will not be appropriately formed, and when it is more than 10000 cells/200 µl/well, there is a risk of necrosis due to undernutrition in the embryoid bodies being caused.

Moreover, the number of days from the start of suspension culture of the embryoid bodies employed in the present invention is not particularly limited, and for example, those that are on Days 5 - 9 from the start of suspension culture can be favorably employed.

In the present invention, when an embryoid body is employed for transplantation, all or a part of an embryoid body can be employed for transplantation. An embryoid body can be employed for transplantation as is, or only a part of an embryoid body can also be employed for transplantation. When only a part of an embryoid body is employed for transplantation, it is preferred to employ the surface tissue of an embryoid body. Since the surface layer of an embryoid body is composed of epithelial line cells, by employing the surface tissue of an embryoid body for transplantation, full-thickness skin can be manufactured in a teratoma more efficiently.

In the present invention, the method for separating only the surface tissue from an embryoid body is not particularly limited. For example, the surface tissue of an embryoid body can be physically collected with microsurgery by a syringe under a stereomicroscope.

A "scaffolding material" as used herein refers to materials in general that express and promote various cell functions such as cell adhesion, proliferation, differentiation, activation, movement, migration, and morphological change by the contact of a cell and the material on or inside the material, and is not particularly limited as long as it is favorable when transplanting pluripotent stem cells. For example, a collagen gel can be employed as the scaffolding material, and preferably, type I collagen gel, type III collagen gel, type IV collagen gel, and Matrigel can be employed. By encapsulating pluripotent stem cells in a scaffolding material and then subjecting it to transplantation, pluripotent stem cells are prevented from dissipating in the transplanted tissue and serves as an scaffolding for the tissue to survive, and thus full-thickness skin can be manufactured in a teratoma more efficiently. Moreover, by encapsulating pluripotent stem cells in a scaffolding material and then subjecting it to transplantation, the embryoid body can be transplanted into a collagen gel while retaining the desired configuration. By performing transplantation with the surface tissue of each embryoid body in contact with each other in a collagen gel, full-thickness skin can be manufactured in a teratoma more efficiently.

In the present invention, the method for creating a conjugate comprising "all or a part of an embryoid body" and a scaffolding material is not particularly limited. The "all or a part of an embryoid body" and the scaffolding material may be bound *ex vivo* and then employed for transplantation, or transplantation may be performed by first introducing the scaffolding material *in vivo* and then injecting the "all or a part of an embryoid body" to be bound thereto. Moreover, for example, when a collagen gel is employed as the scaffolding material and bound with all or a part of an embryoid body, a conjugate of a collagen gel and all or a part of an embryoid body can be created by placing an embryoid body in a collagen gel in a sol state and then solidifying.

A "bioactivation substance that may activate the Wnt pathway" as used herein may be e.g. a bioactivation substance that may activate the classical Wnt pathway (also referred to as β catenin pathway), or a bioactivation substance that may activate the non-classical Wnt pathway (planar cell polarity pathway; PCP pathway, also referred to as Ca2+ pathway). Examples of classical bioactivation substances that may activate the Wnt pathway can be e.g. Wnt1, Wnt2, Wnt2b, Wnt3, Wnt3a, Wnt6, Wnt7b, Wnt8a, Wnt8b, Wnt10b, and TGF-β, and examples of non-classical bioactive substances that may activate the Wnt pathway can be e.g. Wnt4, Wnt5a, and Wnt11. The fact that Wnt10b may activate the classical Wnt pathway (β catenin pathway) is described e.g. in Maksim V. Plikus et al. (Science 332, 586 (2011)), the fact that Wnt1, Wnt2, Wnt2b, Wnt3, Wnt3a, Wnt6, Wnt7b, Wnt8a, and Wnt8b may activate the classical Wnt pathway (β catenin pathway) and Wnt4, Wnt5a, and Wnt11 may activate the non-classical Wnt pathway is described e.g. in Kemp et al. (Functional Development and Embryology 1(1), 1-13 (2007)), and the fact that TGF-β may stabilize the expression of β catenin in dermal fibroblasts is described e.g. in Sato (Acta Derm Venereol 2006; 86: 300-307).

In the present invention, the type of animal for transplanting cells is not particularly limited, and any and all animals can be employed for transplantation. Preferably, by employing a non-human animal such as a pig, a cow, a monkey, a baboon, a dog, a cat, a rat, or a mouse for transplantation, ethical problems that arise from transplanting cells to humans can be avoided. More preferably, by employing a non-human immunodeficient animal for transplantation, rejection due to the immune function of a living body can be prevented, and a teratoma can be efficiently created. Moreover, by employing a non-human immunodeficient animal for transplantation, a teratoma derived from the cells of another type of animal can be created in the living body of a non-human immunodeficient animal. For example, by transplanting human-derived pluripotent stem cells to a non-human immunodeficient animal, a human cell-derived teratoma can be created in the living body of a non-human immunodeficient animal.

An "immunodeficient animal" as used herein refers to an animal that is deficient in a part or all of the immune function of a living body. The type of the deficient immune function is not particularly limited, but the animal is preferably one that is deficient in immune functions such that cells or tissues derived from another type of animal transplanted to the living body are not eliminated. For example, in case of an immunodeficient mouse, a SCID mouse, a nude mouse, a NOD mouse, a NOD-SCID mouse, an IL-2Rg knockout mouse, a RAG2 knockout mouse, a NOG mouse, or a RAG2/IL-2Rg double knockout mouse can be employed, and preferably a SCID mouse can be employed. Moreover, for example, in case of an immunodeficient rat, a SCID rat can be employed. Moreover, in case of an immunodeficient pig, an IL-2rg knockout pig can be employed.

In the present invention, the method for resecting the desired organ from a teratoma is not particularly limited, and for example, resection can be performed by microsurgery.

Note that the terms used herein are to be employed to describe particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

Terms such as first and second are sometimes employed to express various elements, and it should be recognized that these elements are not to be limited by these terms. These terms are employed solely for the purpose of discriminating one element from another, and it is for example possible to describe a first element as a second element, and similarly, to describe a second element as a first element without departing from the scope of the present invention.

The present invention will now be more specifically described by Examples. However, the present invention can be embodied by various embodiments, shall not be construed as being limited to the Examples described herein.

### Examples

### Formation of full-thickness skin by in vivo transplantation of conjugate comprising embryoid body

### 1. Materials and methods

### (1) Laboratory animals

C.B-17/lcr-scid/scidJcl mice were purchased from CLEA (Tokyo, Japan), and scid/scid hr/hr (SHO) mice were purchased from Charles River (Kanagawa, Japan). Control and handling of the mice were in compliance with the NIH Laboratory animal guideline. All experiments were carried out under the approval of the laboratory animal control committee at Tokyo University of Science.

### (2) Cell culture

Mouse iPS cells (mGF-iPS-3F-3) were co-cultured with SNLP 76.7-4 feeder cells treated with mitomycin C (Nacalai Tesque). For culture, a medium, Dulbecco's Modified Eagle's Medium (DMEM without sodium pyruvate; Nacalai Tesque) supplemented with 15% fetal bovine serum (Japan Bio Serum), 50 units/ml penicillin, 50 µg/ml streptomycin, 2 mM L-glutamine, 1 x 10⁻⁴ M 2-Mercaptoethanol, and 1 x 10⁻⁴ M Non-essential amino acids (all from Invitrogen) was employed. The medium was exchanged every day, and on Day 2 after subculture, subcultured with a solution of D-PBS (-) (Nacalai Tesque) supplemented with 0.25% Trypsin - 1 mM EDTA (Invitrogen).

SNLP 76.7-4 feeder cells were cultured on a dish that was gelatin-coated with 0.1% gelatin aqueous solution at 37°C for 2 hours or more. For culture, a medium of Dulbecco's Modified Eagle's Medium (DMEM without sodium pyruvate; Nacalai Tesque) supplemented with 7% fetal bovine serum, 50 units/mL penicillin, 50 µg/ml streptomycin, and 2 mM L-glutamine (all from Invitrogen) was employed. Mitomycin C was added to said medium to a final concentration of 12 µg/ml, and SNLP 76.7-4 feeder cells were reacted at 37°C for 2 hours and 15 minutes to perform mitomycin treatment of SNLP 76.7-4 feeder cells. Then, the reacted cells were seeded at 2.5 x 10⁴ cells/cm² in the gelatin-coated dish. Those that were cultured for 24 hours or more after mitomycin treatment were employed as feeder cells for co-culture with iPS cells.

### (3) Embryoid body production

The iPS cells were detached together with the feeder cells from the culture dish by enzyme treatment, and made into single cells by mild pipetting. With a cell sorter (FACS AriaIII, BD), the feeder cells were removed by SSC and FSC to sort only the iPS cells. The sorted iPS cells were suspended in the iPS cell culture medium described in (2) to 1.5 x 10⁴ cells/ml, and further seeded in a 96-well low adherence plate (Lipidure, NOF) at 3,000 cells/200 µL/well.

### (4) Wnt10b stimulation of embryoid body

iPS cells were seeded in a low cell adherence plate by the methods described in (2) and (3), and then cultured with a medium, Iscove's Modified Dulbecco's Medium (IMDM; GIBCO) supplemented with 10% fetal bovine serum (Japan Bio Serum), 50 units/mL penicillin, and 50 µg/ml streptomycin for 7 days. On Day 4 after seeding, half of the medium was exchanged, and on culture Day 7, embryoid body formation was confirmed by a phase contrast microscope and Wnt10b stimulation was performed on embryoid bodies without morphological defect. 0.1 mg/mL Wnt10b (R&D) in PBS as stock was added to the medium for iPS cells to 1 µg/mL. Half of the medium of wells having embryoid bodies formed was discarded, and this half was exchanged with the medium for iPS cells comprising Wnt10b (final concentration 500 ng/mL). Embryoid bodies in the medium supplemented with Wnt10b were cultured for 24 hours in a CO₂ incubator.

### (5) Formation of teratoma by transplantation of iPS cell-derived embryoid body

Thirty-microliter cold type I collagen gel (Nitta Gelatin) drops were formed on a sterile plastic dish with a thin application of silicone grease, 32 or 48 embryoid bodies were quickly incorporated before gel formation, and this was incubated in a CO₂ incubator at 37°C for 10 minutes to gel. The collagen gels incorporating embryoid bodies were transplanted one each to the subrenal capsule of both kidneys of C.B-17/lcr-scid/scidJcl mice (7 - 10 weeks-old) under anesthesia. On Day 28 or 30 after transplantation, the mice having iPS cell-derived embryoid bodies transplanted were sacrificed and the teratomas were resected.

### (6) Histological analysis of cysts in teratoma

In order to perform analysis of cystic epithelium in teratomas and the histological analysis of the induced organ, the weight of the resected teratoma was measured and a macrophotograph was taken, and then the aforementioned teratoma was immersed in Mildform 10N fixing solution (Wako) and fixed overnight at room temperature. The fixed teratoma tissue was paraffin embedded or freeze embedded according to an ordinary method, and serial sections having a thickness of 10 µm were created. All or a part of the serial sections were HE stained with Mayer's hematoxylin to perform histological analysis of cysts in the teratoma. In order to analyze hair follicle formation incidences in the teratoma, a thickness of 3 mm from the block surface was histochemically analyzed, and the number of hair follicles that had formed was counted with an upright microscope Axioimager A1 (Carl Zeiss) and AxioCAM MRc5 (Carl Zeiss). The hair follicles in which the hair bulb portion became the largest on the serial sections were counted. Moreover, tissue images were photographed with an upright microscope Axioimager A1 (Carl Zeiss) and AxioCAM MRc5 (Carl Zeiss).

### 2. Results

### (7) Formation of full-thickness skin and mucosa-like tissue by in vivo transplantation of conjugate comprising embryoid body

### (7-1) Histological analysis of an in vivo transplant of conjugate comprising embryoid body

In regards to cysts in teratomas that were formed by *in vivo* transplantation of a conjugate comprising an embryoid body, HE staining images of serial sections were observed to analyze histological characteristics. As a result, a hair follicle having a sebaceous gland is connected to a skin-like cyst, and with the connection point of the sebaceous gland as the borderline, an eosinophilic dermal layer with fibroblasts dispersed is positioned to the direction of the cystic epithelium, and fat tissues were distributed to the direction of the hair bulb portion. The epidermal layer of the cyst has the basal cell layer, the polar cell layer, the granulosa layer, and the corneal layer regularly arranged, and the corneal layer was shown to be a typical skin epidermal tissue since it detaches in a clearly laminar state towards the inside of the cystic cavity. It was shown that the hair shaft grows from the opening of the hair follicle towards the cyst lumen (Figure 1, black arrows). The connection site of the sebaceous gland and the hair follicle is hair follicle infundibular part or the opening of the pore (Figure 1, left, white arrowheads), and it was suggested that sebum may be secreted via the pores to the outer layer of the epidermis. This structure completely matches the histological characteristics of natural skin, and it was shown that full-thickness skin was induced with the cystic cavity as the center. On the other hand, ectodermal organs such as hair follicles were not observed in the mucosa-like cyst, a somewhat unclear monolayer keratinized epithelial layer is surrounded by lamina propria mucosae and submucosal layer which are loose connective tissues, and a smooth muscle layer and a secretory gland-like tissue are arranged in the submucosal layer, and it was shown to be histologically similar to mucosa etc. (Figure 1, right).

### (7-2) Induction of epithelial tissue region by addition of Wnt10b during embryoid body formation

Tissue sections of a teratoma form in mouse subrenal capsule by the methods described in (3) - (5) were HE stained (Figure 2A), classified into dermatoid (squamous stratified keratinized epithelial layer / dermal layer / fat or striated muscle), mucosa-like (squamous stratified keratinized epithelium / lamina propria mucosae / striated muscle), transitional epithelium (transitional form of squamous stratified keratinized epithelium and monolayer columnar epithelium), or endodermal epithelioid (monolayer columnar epithelium / lamina propria mucosae / striated muscle) according to the histological characteristics of the epithelial tissue and the surrounding interstitial tissue of the cysts formed, and the configuration ratio thereof was measured (Figure 2B). As a result, compared to a transplant by an embryoid body without Wnt10b stimulation, when an embryoid body subjected to Wnt10b stimulation was transplanted, the cysts in the endodermal epithelioid were decreased and incidences of dermatoid and mucosa-like were increased (Figure 2C).

### (7-3) Increase in hair follicle formation incidences by addition of Wnt10b

The number of induced hair follicles contained in 1 g of teratomas derived from embryoid bodies stimulated with Wnt10b stimulation (285 ± 128, n = 4) increased significantly compared to the group not treated with Wnt10b (39 ± 21, n = 8) (Figure 2D). Moreover, when the length of hair follicles grown from hair shafts induced in teratomas at Days 28 - 30 after embryoid body transplantation into the subrenal capsule was measured, the length in the Wnt10b addition group was twice that of the non-treated group.

### (7-4) Induction of exocrine gland tissue by addition of Wnt10b

In the HE tissue analysis of teratomas derived from embryoid bodies with Wnt10b stimulation, a structure where numerous acinar structures of the exocrine gland have aggregated was observed (Figure 1, right). Accordingly, when a tissue similar to the acinus of the exocrine gland was immunostained with antibodies against an amylase secreted only by the salivary gland and the pancreatic exocrine gland, a granular positive image characteristic of secretory vesicles was shown in the cytoplasm of cells positive against an epithelial cell marker E cadherin. From this result, it was suggested that not only hair follicles but also exocrine glands such as the salivary gland were induced by Wnt10b stimulation (Figure 3).

### (8) Evaluation of quality and function of iPS cell-derived hair follicle and full-thickness skin

### (8-1) Trichogenic ability of iPS-induced hair due to transplantation into skin

In order to investigate whether a hair follicle organ contained in full-thickness skin induced in a teratoma is fully functional and transplantable, full-thickness skin comprising hair follicles induced by *in vivo* transplantation of iPS cells was resected, cut up into hair groups, and transplanted into nude mouse skin. It became clear that the transplanted hair group shedded on Day 7 after transplantation into skin, and thereafter, hair grew on Day 14 at an incidence of 66% (77 out of 117 cases) (Figure 4). It was shown that iPS cell-derived hair follicles and skin tissue reproduce the function of being transplantable to the skin of a living body.

### (8-2) Hair cycle analysis of iPS-induced hair

In order to investigate whether a hair follicle organ contained in full-thickness skin induced in a teratoma repeats the hair cycle and is permanently functional, full-thickness skin comprising hair follicles induced by *in vivo* transplantation of iPS cells was resected, cut up into hair groups, and transplanted into nude mouse skin (in the present specification, hair follicles induced from iPS cells by the method of the present invention are referred to as "iPS-induced hair follicles", and hair induced by transplantation of an iPS-induced hair follicle is referred to as an "iPS-induced hair".) The hair type of the hair shafts grown from the group subjected to said transplantation was distinguished, and it was found to contain Zigzag, Awl, and Guard hair included as body hair. Accordingly, when the growth of hair shafts according to hair type was tracked to the third hair cycle and the length of the hair shaft growth period as well as the hair shaft growth rest and shedding periods were analyzed, it was shown that they repeat the cyclicity similar to that of adult body hair (Figure 5). From these results, it was suggested that iPS-induced hair reproduces the stem cell niche of a living body and permanently repeats the hair cycle.

### (8-3) Analysis of origin of iPS-induced hair

In order to prove that the full-thickness skin transplanted in the method described in (8-2) and the hairs grown are derived from iPS cells, Y chromosomes were labeled and fluorescent *in situ* hybridization (FISH) was performed. Since the iPS cells used in the present experiments are cells derived from a male mouse and the transplanted Balb/c nu/nu mice are female mice, Y-chromosomes in the nucleus were labeled with green fluorescent dye and the origin of the organs (full-thickness skin and hair follicles grown) produced from the transplant were analyzed. As a result, it became clear that full-thickness skin comprising skin appendage such as hair follicles are composed of Y-chromosome-positive cells, i.e. cells induced from an iPS cell (Figure 6). Moreover, while the epithelial layer connected to the hair follicle is Y-chromosome-positive, Y-chromosomes were not detected in the host tissue.

### (8-4) Niche analysis of iPS-induced hair

To clarify whether induced hair contained in full-thickness skin induced from iPS cells forms a stem cell niche, immunostaining was performed with epithelium stem cell markers CD34 and CK15. Moreover, in order to clarify whether the epithelial stem cell niche committed to variable regions or the sebaceous gland and the skin epidermis are reproduced, the behavior of Lgr5, Lgr6, and Lrig1-positive cells were analyzed. Since the bulge region that is histologically defined as the bulged outer root sheath on the lower side of the sebaceous gland functions as the stem cell niche where hair follicle epithelium stem cells positive both to CD34 and CK15 are localized and is essential for maintaining hair follicle homeostasis, iPS-induced hair follicles were analyzed by immunostaining with these as markers. As a result, when CD34 was fluorescently labeled with red and CK15 with green, the outer root sheath that histologically corresponds to the bulge region of iPS-induced hair follicles was stained yellow (Figure 7b, arrow). From this, it was shown that the epithelium stem cells that co-express CD34 and CK15 were stored in the bulge region. Accordingly, it was shown that iPS-induced hair constructs the epithelial stem cell niche. It is known that in a growing phase hair follicle, Lgr5-positive cells which are hair matrix progenitor cells are distributed below the outer root sheath cell of the variable region of the hair follicle and the critical line of Auber of the hair matrix, and in a resting phase hair follicle, Lgr5-positive cells are localized in secondary hair buds. Moreover, Lgr6 and Lrig1-positive cells are localized from the upper part of the bulge near the sebaceous gland attachment site. When the behavior of Lgr5, Lgr6, and Lrig1-positive cells were also analyzed in the growing phase and the resting phase of iPS-induced hair follicles, expression was confirm at the same site as natural hair for each of Lgr5, Lgr6, and Lrig1 (Figure 7).

### (8-5) Connection between iPS-induced hair follicle and surrounding tissue

In order to determine whether an iPS-induced hair follicle is connected to the arrector pili muscle and the nerve, sections with a thickness of 100 µm were created, immunostaining was performed with antibodies against a nerve fiber marker neurofilament, a smooth muscle marker calponin, and a striated muscle marker troponin, and analyzed with a confocal laser microscope. In a natural body hair, arrector pili muscle consisting of calponin-positive smooth muscle is connected to the bulge region. To this, a sympathetic nerve extending from deep plexus is arranged to surround the arrector pili muscle, thereby forming the neuromuscular connection site to receive nerve control. Since iPS-induced hair follicle is a colored hair and can be distinguished from a host hair follicle, iPS-induced hair follicles were distinguished from host hair follicles, and then nerve fiber and arrector pili muscle connections were analyzed by immunostaining. As a result, similarly to natural body hair, calponin-positive arrector pili muscle was connected to the bulge region (Figure 8, arrows), and nerve fibers were connected thereto (Figure 8, arrowheads). Further, nerve-hair follicle connection sites were seen not only in the connection between the hair follicle and the arrector pili muscle but also the subbulge epithelium (Figure 8). Nerve fiber connection was seen around the bulge region, the nerve terminal was distributed in the ORS outermost layer of the bulge region, and nerve-hair follicle connection sites were seen (Figure 8).

### (8-6) Tumorigenicity assay by nude mouse subcutaneous transplantation of single iPS cells and full-thickness skin comprising iPS-induced hair follicles

To test whether transplantation of full-thickness skin induced from iPS cells will form a tumor, full-thickness skin comprising corresponding to 20 hair follicles was transplanted to the dorsal skin of nude mice, and tumor mass formation due to proliferation of tumor cells was tracked over three months. As a comparison group, iPS cells that were made into single cells from the same iPS cell line were created, intradermally transplanted at 1 x 10⁴, 1 x 10⁵, and 1 x 10⁶ cells, and tracked for the same duration. As a result, in the single cells transplantations, increase in tumor mass due to tumor formation was seen in any number of cells, tumor formation was seen at 20 to 40 days after transplantation, and tumor formation was prone to be faster depending on the number of transplanted cells (Table 1). In contrast, in the transplantation of iPS-induced hair follicles, increase in tumor mass due to tumor formation was not observed in a tracking up to 90 days after transplantation (Table 1).
[Table 1]

**Table 1. Tumorigenicity by nude mouse intradermal transplantation of iPS-induced hair follicles and single iPS cells**

| Transplant | Transplantation condition | Tumor formation incidence | Number of days for tumor formation (mean ± se) |
|---|---|---|---|
| Single iPS cells | 10,000 cells | 17% (n=6) | 44.0 |
| | 100,000 cells | 33% (n=6) | 34.5 ± 1.5 |
| | 1,000,000 cells | 50% (n=6) | 20.3 ± 1.9 |
| iPS-induced hair group | Corresponds to 20 HFs | ND (n=18) | ND |

From the above results, according to the method of the present invention, full-thickness skin with skin appendage can be artificially manufactured efficiently. Moreover, it was shown that the full-thickness skin manufactured by the method of the present invention has extremely low risk of causing a tumor by transplantation, and is extremely promising also as an organ formation technology with premises of transplantation to a living body.

## Claims

1. A method for manufacturing full-thickness skin with skin appendage, **characterized in that**
said "full-thickness skin with skin appendage" comprises at least the following (1) - (3):
(1) skin comprising epidermal and dermal layers,
(2) at least one type of skin appendage, and
(3) subcutaneous tissue,
wherein said method comprises the following steps:
(a) a step of stimulating an embryoid body with a bioactive substance that may activate the Wnt pathway,
(b) a step of preparing a conjugate comprising the following (A) and (B):
(A) all or a part of said embryoid body stimulated in step (a) and
(B) a scaffolding material
(c) a step of transplanting said conjugate prepared in said step (b) to an animal, and
(d) a step of manufacturing full-thickness skin derived from said conjugate in said animal.

2. The method according to claim 1, wherein said animal is a non-human animal.

3. The method according to claim 2, wherein said non-human animal is a non-human immunodeficient animal.

4. The method according to claim 2 or 3, wherein said Wnt pathway is the classical Wnt pathway.

5. The method according to claim 2 or 3, wherein said "bioactive substance that may activate the Wnt pathway" is selected from a group consisting of Wnt1, Wnt2, Wnt2b, Wnt3, Wnt3a, Wnt6, Wnt7b, Wnt8a, Wnt8b, Wnt10b, and TGF-β.

6. The method according to claim 2 or 3, wherein said embryoid body is an embryoid body created from an iPS or ES cell.

7. The method according to claim 2 or 3, wherein said scaffolding material is a collagen gel.

8. The method according to claim 2 or 3, wherein said transplantation is transplantation to the subrenal capsule.

9. A full-thickness skin with skin appendage manufactured by the method according to any one of claims 1 to 8.
